(19) Europäisches Patentamt
European Patent Office
Office européen des brevets

(11) Numéro de publication : **0 082 058**
**B1**

(12) **FASCICULE DE BREVET EUROPÉEN**

(45) Date de publication du fascicule du brevet : **07.08.85**

(51) Int. Cl.⁴ : **C 07 D 213/53**, A 61 K 31/44

(21) Numéro de dépôt : **82402222.2**

(22) Date de dépôt : **06.12.82**

(54) **Ethers d'oximes de pyridyl-1 pentanone-3, leur procédé de préparation et leur utilisation comme médicament.**

(30) Priorité : **11.12.81 FR 8123147**

(43) Date de publication de la demande : **22.06.83 Bulletin 83/25**

(45) Mention de la délivrance du brevet : **07.08.85 Bulletin 85/32**

(84) Etats contractants désignés : **CH DE FR GB IT LI**

(56) Documents cités :
**EP-A- 0 007 679**
**FR-A- 1 217 007**
**FR-A- 2 253 505**

(73) Titulaire : **UNIVABLOT**
**21 bis rue Jonquoy**
**F-75014 Paris (FR)**

(72) Inventeur : **Astoin, Jacques Noel**
**5 rue Basse des Carmes**
**F-75005 Paris (FR)**
Inventeur : **Lepage, Francis**
**13 rue du Général de Larminat**
**F-94000 Creteil (FR)**
Inventeur : **Fromantin, Jean-Pierre Marie Joseph**
**50 avenue Villeneuve l'Etang**
**F-78000 Versailles (FR)**

(74) Mandataire : **Lepeudry-Gautherat, Thérèse et al**
**CABINET ARMENGAUD JEUNE CASANOVA et LEPEUDRY 23 boulevard de Strasbourg**
**F-75010 Paris (FR)**

**EP 0 082 058 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des éthers d'oximes de pyridyl-1 pentanone-3, leur procédé de préparation et leur utilisation comme médicament.

Les composés selon l'invention répondent à la formule générale (I)

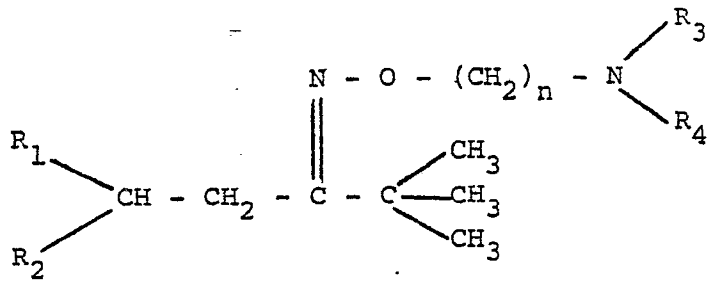

(I)

dans laquelle

$R_1$ est choisi parmi un radical cyclohexyle, un radical phényle ou benzyle éventuellement substitué par un halogène ou par un radical alkyle linéaire ou ramifié ayant de 1 à 4 atomes de carbone,

$R_2$ est un radical pyridyle,

$R_3$ et $R_4$ identiques ou différents représentent un radical alkyle linéaire ou ramifié ayant de 1 à 4 atomes de carbone, ou bien $R_3$ et $R_4$ représentent conjointement une chaîne alkylène en $C_4$-$C_7$ pouvant également éventuellement contenir un hétéro-atome d'oxygène ou d'azote,

n est un nombre entier compris entre 1 et 4,

ainsi que les sels, les dérivés d'ammonium quaternaire, les stéréo-isomères possibles et leur mélange, desdits éthers oximes.

Les composés préférés sont ceux pour lesquels n = 2 ou 3. Plus particulièrement les composés préférés sont ceux pour lesquels n = 2 ou 3 et $R_3$ et $R_4$ représentent conjointement une chaîne alkylène en $C_5$ et $R_1$ est un radical phényle.

Les éthers d'oximes selon l'invention comprennent également tous leurs stéréo-isomères possibles et les mélanges de ceux-ci.

Les composés de formule (I) peuvent être préparés par les procédés suivants :

Selon un premier procédé, on fait réagir une cétone de formule générale (II)

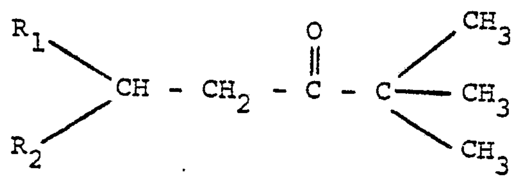

(II)

avec un dérivé d'hydroxylamine de formule générale (III)

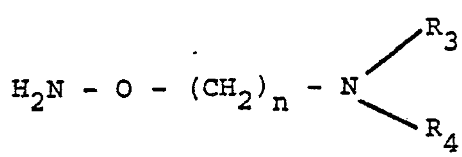

(III)

dans lesquelles $R_1$, $R_2$, $R_3$, $R_4$ et n ont les significations données ci-dessus, en présence de préférence, d'un solvant inerte vis-à-vis de la réaction, par exemple l'éthanol ou la pyridine.

Selon un deuxième procédé, on fait réagir une oxime de formule générale (IV)

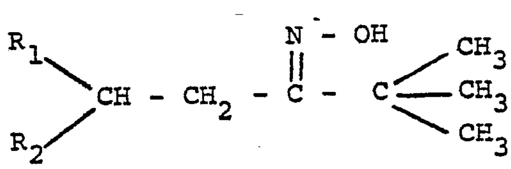

(IV)

avec un dérivé chloré d'alkylamine de formule (V)

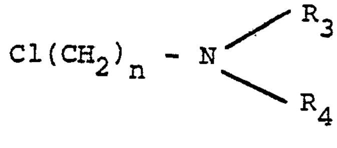

(V)

dans lesquelles $R_1$, $R_2$, $R_3$, $R_4$ et n ont les significations données ci-dessus. La réaction a lieu en présence d'un solvant inerte tel que par exemple le diméthylformamide, le toluène ou le cumène en présence d'un agent de condensation basique tel que des hydrures ou des amides de métaux alcalins tel que l'hydrure de sodium.

Les cétones de formule (II) peuvent être obtenues par action d'un organomagnésien de formule (VI)

$$R_1Mg\ Hal \qquad (VI)$$

sur des cétones de formule (VII)

$$R_2—CH = CH—\overset{\overset{O}{\|}}{C}—C(CH_3)_3 \qquad (VII)$$

Les cétones de formule (VII) dont certaines sont par exemple décrites dans le brevet français 73 43 940 publié sous le N° 2 253 505 peuvent être obtenues en condensant en milieux aqueux ou alcoolique la pinacoline (diméthyl-3,3 butanone) avec un aldéhyde de formule (VIII)

$$R_2—CHO \qquad (VIII)$$

Dans les formules (VI) à (VIII) $R_1$ et $R_2$ ont la signification mentionnée ci-dessus et Hal désigne un halogène.

Les composés de formule I peuvent être transformés de manière connue en leurs sels d'addition avec un acide ou en leurs sels d'ammonium quaternaire.

On prépare les sels d'addition d'acide avec des acides physiologiquement acceptables tels que l'acide citrique, l'acide fumarique, l'acide acétique etc...

Pour préparer les sels d'ammonium quaternaire l'on fait réagir des composés de formule générale (I) avec des composés propres à se transformer en dérivés quaternaires tels que par exemple un halogénure d'alkyle ou un ester d'acide méthane-sulfonique.

Les composés selon l'invention ainsi que leurs sels d'addition physiologiquement acceptables sont utilisables comme médicaments notamment pour leur action spasmolytique et antihistaminique, par exemple pour le traitement des maladies cardiovasculaires.

Les composés selon l'invention peuvent être présentés en association avec un excipient pharmaceutiquement compatible pour l'administration par voie orale par exemple sous forme de comprimés, dragées ou gélules et pour l'administration par voie parentérale sous forme de solutions injectables pour les composés solubles ou pour l'administration endorectale, sous forme de suppositoires.

La posologie quotidienne est de l'ordre de 50 à 250 mg par voie orale et d'environ, 5 à 50 mg par voie injectable.

Pour mettre en évidence l'effet spasmolytique, on utilise la méthode de MAGNUS (Versuche am überbundenen Dünndarm von Säugetieren. Arch. Ges. Physiol. 1904, 102-123). On mesure l'activité antispasmodique sur le spasme provoqué par l'acétylcholine ou le chlorure de baryum, au niveau du duodénum isolé du rat.

Un rat est sacrifié, après ouverture de l'abdomen la portion duodénale est prélevée et sa lumière est soigneusement lavée avec une solution de Tyrode à 37 °C. Le fragment d'intestin est fixé dans un bain à organe isolé à 37 °C et oxygéné. On recherche la dose d'acétylcholine ou de chlorure de baryum exprimée en mole par litre qui additionnée au bain provoque une réponse contractile de l'organe fournissant un tracé sur papier de l'enregistreur. Après lavage de l'organe on recherche la dose de produit selon l'invention qui additionnée au bain durant une minute avant la dose d'acétylcholine ou de chlorure de baryum définie, réduit le spasme de moitié. On opère de la même façon avec un produit de référence l'atropine pour l'acétylcholine et la papavérine pour le chlorure de baryum. On exprime ainsi pour le produit à étudier et le produit de référence, la dose efficace 50.

On met en évidence l'action anti-histaminique de la même façon en utilisant l'ilion isolé de cobaye. La méthode est rigoureusement la même, seul l'agent contracturant change. Il est pour cet essai, l'histamine et le produit de référence est la prométhazine.

D'autres avantages et caractéristiques de l'invention apparaîtront à la lecture des exemples suivants donnés à titre illustratif.

## Exemple 1

Phényl-1 (pyridyl-2)-1 diméthyl-4,4 pentanone-3

On ajoute goutte à goutte une suspension éthérée de 0,13 mole (pyridyl-2)-1 diméthyl-4,4 pentène-1 one-3 dans une solution de phénylbromomagnésien préparée à partir de 0,17 mole de bromobenzène, 0,17 atome-gramme de magnésien dans 150 ml d'éther. On porte au reflux 1/2 h. On décompose le produit obtenu dans de l'eau acidulée, on extrait à l'éther, on sèche puis on concentre et on recristallise dans l'éther de pétrole. Le rendement est de 87 %. On prépare la phényl-1 (pyridyl-2)-1 diméthyl-4,4 pentanone-3

oxime correspondante par réaction de 0,1 mole de chlorhydrate d'hydroxylamine sur 0,1 mole de cétone en présence de 0,05 mole de carbonate de sodium.

### Exemples 2 à 6

On prépare les cétones de départ de formule générale (II) et les oximes correspondantes de formule générale (IV) selon une méthode analogue à celle décrite dans l'exemple 1. Les formules particulières de ces cétones ainsi que les oximes correspondantes de formule générale (IV) sont indiquées avec leur constante physique dans le tableau I ci-dessous.

Tous les composés 1 à 6 sont rassemblés dans le tableau I ci-après avec leurs constantes physiques.

Tableau I

| Ex. | Formules (II) ou (V) $R_1$ | $R_2$ | Pt d'ébullition Eb : °C/mm Hg et/ou Pt de fusion F : °C — Cétones de formule (II) | Oximes de formule (IV) |
|---|---|---|---|---|
| 1 | 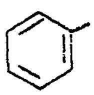 | 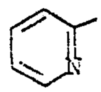 | Eb : 138/0,03 | F : 158 |
| 2 | 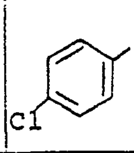 | 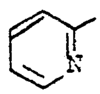 | Eb : 174/0,12 | F : 92 |
| 3 | 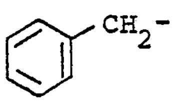 | 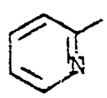 | Eb : 147/0,05 | F : 112 |
| 4 | 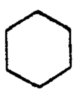 | 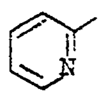 | Eb : 154/0,05 | F : 124 |
| 5 | 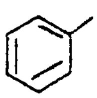 | 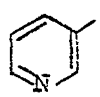 | Eb : 156/0,05<br>F : 82 | F : 115 |
| 6 | 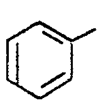 | 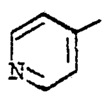 | Eb : 163/0,08<br>F : 78 | F : 142 |

### Exemple 7

Ether O-(diéthylaminoéthylique) de l'oxime de la phényl-1 (pyridyl-2)-1 diméthyl-4,4 pentanone-3.

1er procédé

On porte au reflux pendant 3 heures, 0,1 mole de la cétone de l'exemple 1, 0,1 mole de chlorhydrate de diéthylamino-éthoxyamine en solution dans 150 ml d'éthanol anhydre en présence de 75 ml de pyridine. On concentre, on alcalinise et on extrait au benzène. Le rendement est de 65 %.

2ème procédé

On ajoute lentement 1,4 g d'hydrure de sodium à 50 %, dans l'huile à une solution de 0,03 mole de

l'oxime de l'exemple 1 dans du diméthylformamide. On ajoute ensuite 0,03 mole de chloro-2 éthyldiéthylamine. Après 6 heures d'agitation à 70 °C, on verse la solution sur de l'eau. On extrait à l'éther, on sèche et on concentre, le rendement est de 51 %. La formule, les constantes physiques et les résultats des essais sur l'effet spasmolytique et anti-hystaminique du produit obtenu sont indiqués dans le tableau II ci-dessous.

Exemples 8 à 18

On opère suivant le procédé de l'exemple 7, en partant des cétones ou des oximes des exemples 1 à 6 et des dérivés d'amino-alcoxyamine correspondants. Les formules, les constantes physiques et les résultats des essais sur l'effet spasmolytique et anti-hystaminique sont indiqués dans le tableau II ci-dessous.

Les produits synthétisés sont :

Exemple 8 : l'éther O-(diméthylaminoéthylique) de l'oxime de la phényl-1 (pyridyl-2)-1 diméthyl-4,4 pentanone-3,

Exemple 9 : l'éther O-(diisopropylaminoéthylique) de l'oxime de la phényl-1 (pyridyl-2)-1 diméthyl-4,4 pentanone-3,

Exemple 10 : l'éther O-(pyrrolidinoéthylique) de l'oxime de la phényl-1 (pyridyl-2)-1 diméthyl-4,4 pentanone-3,

Exemple 11 : l'éther O-(diméthylaminopropylique) de l'oxime de la phényl-1 (pyridyl-2)-1 diméthyl-4,4 pentanone-3,

Exemple 12 : l'éther O-(diéthylaminoéthylique) de l'oxime de la (chloro-4 phényl)-1 (pyridyl-2)-1 diméthyl-4,4 pentanone-3.

Exemple 13 : l'éther O-(N-morpholinoéthylique) de l'oxime de la phényl-1 (pyridyl-2)-1 diméthyl-4,4 pentanone-3.

Exemple 14 : l'éther O-(N-méthylpipérazinoéthylique) de l'oxime de la phényl-1 (pyridyl-2)-1 diméthyl-4,4 pentanone-3.

Exemple 15 : l'éther O-(diéthylaminoéthylique) de l'oxime de la cyclohexyl-1 (pyridyl-2)-1 diméthyl-4,4 pentanone-3.

Exemple 16 : l'éther O-(diéthylaminoéthylique) de l'oxime de la benzyl-1 (pyridyl-2)-1 diméthyl-4,4 pentanone-3.

Exemple 17 : l'éther O-(diéthylaminoéthylique) de l'oxime de la phényl-1 (pyridyl-3)-1 diméthyl-4,4 pentanone-3.

Exemple 18 : l'éther O-(diéthylaminoéthylique) de l'oxime de la phényl-1 (pyridyl-4)-1 diméthyl-4,4 pentanone-3.

Des résultats des essais pharmacologiques du tableau II, il apparaît que ces composés présentent une activité intéressante.

(Voir tableau II pages suivantes)

Tableau II

| Ex. | Formule générale (I) | | | | n | Point d'ébullition Eb : °C/mmHg et/ou point de fusion chlorhydrate F : °C | Acétylcholine | | $BaCl_2$ | | Histamine | |
|---|---|---|---|---|---|---|---|---|---|---|---|---|
| | $R_1$ | $R_2$ | $R_3$ | $R_4$ | | | $C_1$ | $DE_{50}$ | $C_2$ | $DE_{50}$ | $C_3$ | $DE_{50}$ |
| 7 | 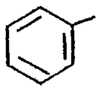 | 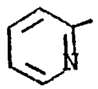 | $CH_3CH_2$- | $CH_3CH_2$- | 2 | 182 /0,15<br>F : 110 | $5.10^{-8}$ | 49 | $5.10^{-7}$ | 66 | $5.10^{-7}$ | 46 |
| 8 | 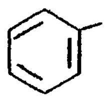 | 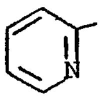 | $CH_3$- | $CH_3$- | 2 | F : 170 | $5.10^{-9}$<br>$10^{-8}$ | 31<br>50 | | | $10^{-6}$ | 50 |
| 9 | 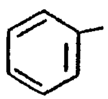 | 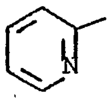 | 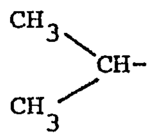 | 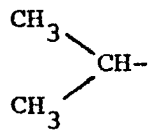 | 2 | 178/0,1 | $10^{-7}$ | 52 | $10^{-6}$ | 65 | $10^{-6}$ | 65 |
| 10 | 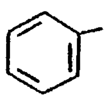 | 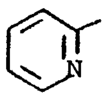 | 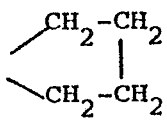 | | 2 | 196/0,2<br>F : 174 | $10^{-8}$ | 52 | $10^{-6}$ | 45 | $5.10^{-6}$ | 67 |
| 11 | 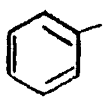 | 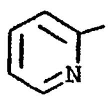 | $CH_3$- | $CH_3$ - | 3 | 172/0,5 | $10^{-7}$ | 13 | $10^{-6}$ | 58 | $10^{-6}$ | 58 |
| 12 | 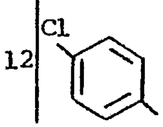 | 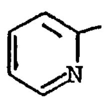 | $CH_3CH_2$- | $CH_3CH_2$- | 2 | 190/0,08 | $5.10^{-7}$ | 44 | | | $10^{-6}$ | 42 |

Tableau II (Suite)

| Ex. | Formule générale (I) | | | | n | Point d'ébullition °C/mmHg et/ou pt de fusion chlorhydrate F : °C | Acétylcholine | | $BaCl_2$ | | Histamine | |
|---|---|---|---|---|---|---|---|---|---|---|---|---|
| | $R_1$ | $R_2$ | $R_3$ | $R_4$ | | | $C_1$ | $DE_{50}$ | $C_2$ | $DE_{50}$ | $C_3$ | $DE_{50}$ |
| 13 | 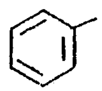 | 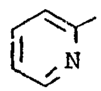 | | 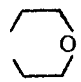 | 2 | 187/0,1 | $10^{-7}$ | 21 | | | $10^{-6}$ | 48 |
| 14 | 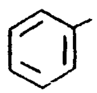 | 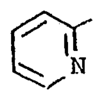 | | 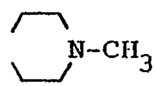 | 2 | 192/0,07 | $10^{-6}$ | 41 | | | $10^{-6}$ | 43 |
| 15 | 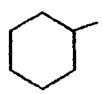 | 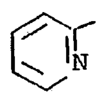 | $CH_3CH_2-$ | $CH_3CH_2-$ | 2 | 164/0,05 | $10^{-7}$<br>$10^{-6}$ | 12<br>73 | | | $10^{-6}$ | 47 |
| 16 | 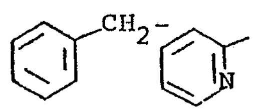 | | $CH_3CH_2-$ | $CH_3CH_2-$ | 2 | 175/0,4 | $10^{-7}$<br>$10^{-6}$ | 18<br>64 | | | $10^{-5}$ | 73 |
| 17 | 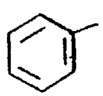 | 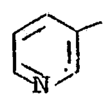 | $CH_3CH_2-$ | $CH_3CH_2-$ | 2 | 162/0,025 | $10^{-7}$<br>$10^{-6}$ | 15<br>73 | | | $10^{-6}$ | 42 |
| 18 | 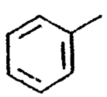 | 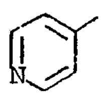 | $CH_3CH_2-$ | $CH_3CH_2-$ | 2 | 168/0,04 | $10^{-6}$ | 40 | | | $10^{-6}$<br>$5.10^{-6}$ | 22<br>95 |

Légende : $C_1$, $C_2$, $C_3$ : concentration en mole/l respectivement en acétylcholine, chlorure de Baryum et Histamine provoquant une contraction de l'organe.
$DE_{50}$ : Dose efficace 50.

**Revendications**

1. Ethers d'oximes, caractérisés en ce qu'ils répondent à la formule générale (I)

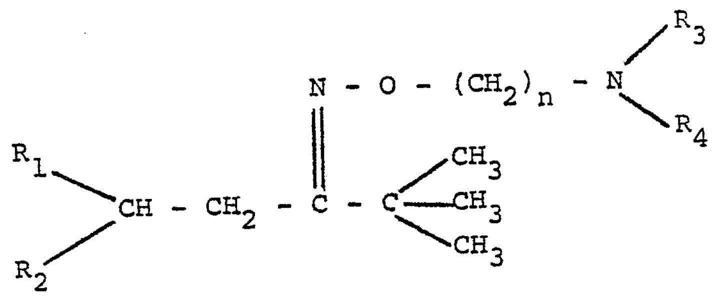

(I)

dans laquelle

$R_1$ est choisi parmi un radical cyclohexyle, un radical phényle ou benzyle éventuellement substitué par un halogène ou par un radical alkyle linéaire ou ramifié ayant de 1 à 4 atomes de carbone,

$R_2$ est un radical pyridyle,

$R_3$ et $R_4$ identiques ou différents représentent un radical alkyle, linéaire ou ramifié ayant de 1 à 4 atomes de carbone, ou bien $R_3$ et $R_4$ représentent conjointement une chaîne alkylène en $C_4$-$C_7$ pouvant également éventuellement contenir un hétéroatome d'oxygène ou d'azote,

n est un nombre entier compris entre 1 et 4, ainsi que les sels, les dérivés d'ammonium quaternaire, les stéréo-isomères possibles et leur mélange, desdits éthers d'oximes.

2. Ethers d'oximes selon la revendication 1, caractérisés en ce que n = 2 ou 3.

3. L'éther O-(pyrrolidinoéthylique) de l'oxime de la phényl-1 (pyridyl-2)-1 diméthyl-4,4 pentanone-3.

4. L'éther O-(diéthylaminoéthylique) de l'oxime de la phényl-1 (pyridyl-2)-1 diméthyl-4,4 pentanone-3.

5. L'éther O-(diméthylaminopropylique) de l'oxime de la phényl-1 (pyridyl-2)-1 diméthyl-4,4 pentanone-3.

6. L'éther O-(diméthylaminoéthylique) de l'oxime de la phényl-1 (pyridyl-2)-1 diméthyl-4,4 pentanone-3.

7. Procédé de préparation d'éthers d'oximes de formule générale (I) selon la revendication 1, caractérisé en ce que l'on fait réagir une cétone de formule générale (II) ci-après

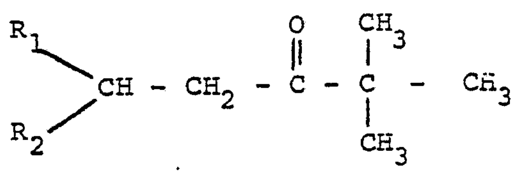

(II)

avec un dérivé d'hydroxylamine de formule générale (III)

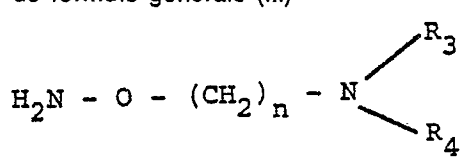

(III)

dans lesquelles $R_1$, $R_2$, $R_3$, $R_4$ et n ont la même signification que ci-dessus.

8. Procédé de préparation d'éthers d'oximes de formule générale (I) selon la revendication 1, caractérisé en ce que l'on fait réagir une oxime de formule générale (IV) ci-après

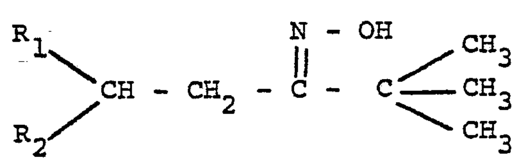

(IV)

avec un dérivé chloré d'alkylamine de formule générale (V)

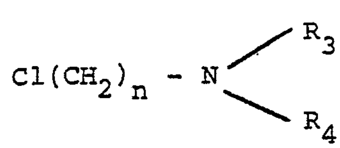

(V)

dans lesquelles $R_1$, $R_2$, $R_3$, $R_4$ et n ont la même signification que ci-dessus, dans un solvant inerte en présence d'un agent de condensation basique.

9. Médicament, caractérisé en ce qu'il comporte comme principe actif une quantité physiologiquement efficace d'au moins un composé selon l'une quelconque des revendications 1 à 6.

10. Composition pharmaceutique comportant un médicament selon la revendication 9, associé à un véhicule pharmaceutique acceptable.

**Claims**

1. Ethers of oximes, characterized in that they respond to general formula (I)

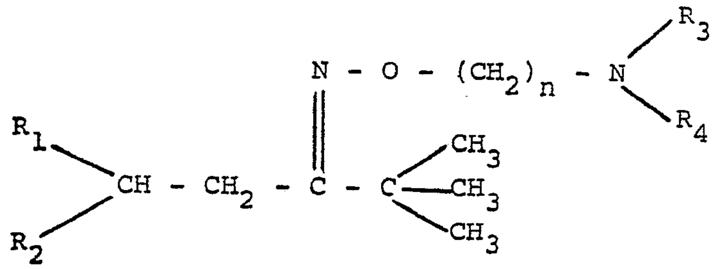

(I)

in which

$R_1$ is selected from a cyclohexyl radical, a phenyl or benzyl radical possibly substituted by a halogen or by a straight or branched alkyl radical having from 1 to 4 atoms of carbon,

$R_2$ is a pyridyl radical,

$R_3$ and $R_4$, identical or different, represent a straight or branched alkyl radical having from 1 to 4 atoms of carbon, or $R_3$ and $R_4$ jointly represent an alkylene chain with $C_4$-$C_7$ which may also possibly contain a heteroatom of oxygen or of nitrogen,

n is an integer included between 1 and 4, as well as the salts, quaternary ammonium derivatives, possible stereo-isomers and their mixture, of said ethers of oximes.

2. Ethers of oximes according to Claim 1, characterized in that n = 2 or 3.

3. The O-(pyrrolidinoethylic) ether of the oxime of 1-phenyl 1-(2-pyridyl) 4,4-dimethyl 3-pentanone.

4. The O-(diethylaminoethylic ether of the oxime of 1-phenyl 1-(2-pyridyl) 4,4-dimethyl 3-pentanone.

5. The O-(dimethylaminopropylic) ether of the oxime of 1-phenyl 1-(2-pyridyl) 4,4-dimethyl 3-pentanone.

6. The O-(dimethylaminoethylic) ether of the oxime of 1-phenyl 1-(2-pyridyl) 4,4-dimethyl 3-pentanone.

7. Process for preparing ethers of oximes of general formula (I) according to Claim 1, characterized in that a ketone of general formule (II) hereinafter

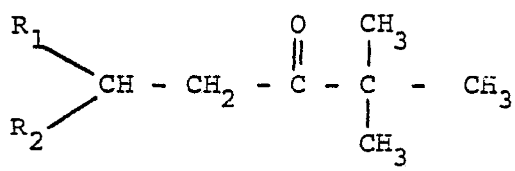

(II)

is reacted with a derivative of hydroxylamine of general formula (III)

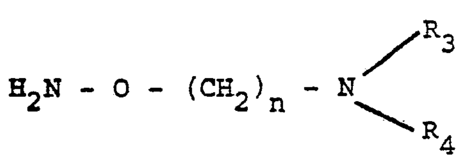

(III)

in which $R_1$, $R_2$, $R_3$, $R_4$ and n have the same meaning as hereinabove.

8. Process for preparing ethers of oximes of general formula (I) according to Claim 1, characterized in that an oxime of general formula (IV) hereinafter

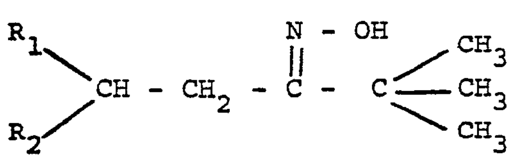

(IV)

is reacted with a chlorinated derivative of alkylamine of general formula (V)

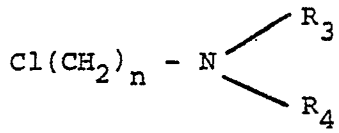

(V)

in which $R_1$, $R_2$, $R_3$, $R_4$ and n have the same meaning as hereinabove, in an inert solvent in the presence of a basic condensation agent.

9. Drug, characterized in that it comprises as active ingredient a physiologically effective quantity of at least one compound according to any one of Claims 1 to 6.

10. Pharmaceutical composition comprising a drug according to Claim 9, associated with a pharmaceutically acceptable vehicle.

**Patentansprüche**

1. Äther von Oximen, dadurch gekennzeichnet, daß sie der allgemeinen Formel (I) entsprechen

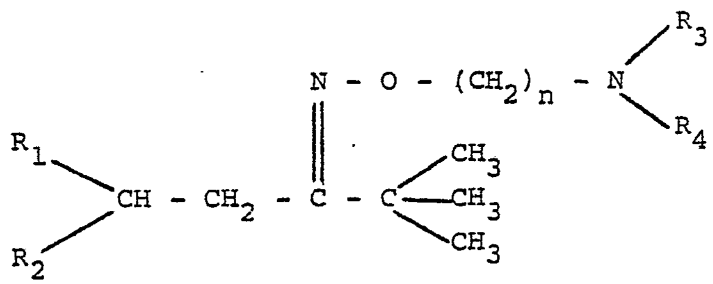

(I)

in welcher

$R_1$ ein Cyclohexyl-, ein Phenyl- oder ein Benzyl-Rest ist, die gegebenenfalls substituiert sind durch ein Halogenatom oder durch einen gerad- oder verzweigtkettigen Alkylrest mit 1 bis 4 Kohlenstoffatomen,

$R_2$ ein Pyridylrest ist,

$R_3$ und $R_4$, die identisch oder voneinander verschieden sein können, einen gerad- oder verzweigtkettigen Alkylrest mit 1 bis 4 Kohlenstoffatomen darstellen oder gemeinsam eine einen Ring bildende Alkylenkette mit 4 bis 7 Kohlenstoffatomen darstellen, die gegebenenfalls ein Sauerstoff- oder Stickstoff-Heteroatom aufweist, und

n eine ganze Zahl von 1 bis 4 ist, einschließlich ihrer Salze, ihrer quaternären Ammoniumverbindungen, ihrer möglichen Stereoisomeren und deren Gemisches.

2. Äther von Oximen nach Anspruch 1, dadurch gekennzeichnet, daß n = 2 oder 3.

3. Der O-(Pyrrolidinoethyl)-Äther des Oxims von Phenyl-1 (pyridyl-2)-1 dimethyl-4,4 pentanon-3.

4. Der O-(Diethylaminoethyl)-Äther des Oxims von Phenyl-1 (pyridyl-2)-1 dimethyl-4,4 pentanon-3.

5. Der O-(Dimethylaminopropyl)-Äther des Oxims von Phenyl-1 (pyridyl-2)-1 dimethyl-4,4 pentanon-3.

6. Der O-(Dimethylaminoethyl)-Äther des Oxims von Phenyl-1 (pyridyl-2)-1 dimethyl-4,4 pentanon-3.

7. Verfahren zur Herstellung von Äthern von Oximen der allgemeinen Formel (I) gemäß Anspruch 1, dadurch gekennzeichnet, daß man ein Keton der allgemeinen Formel (II)

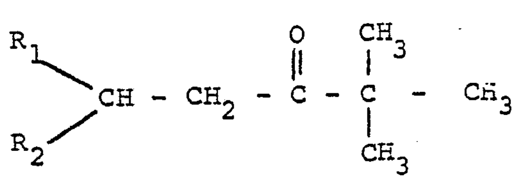

(II)

mit einem Hydroxylaminderivat der allgemeinen Formel (III) umsetzt,

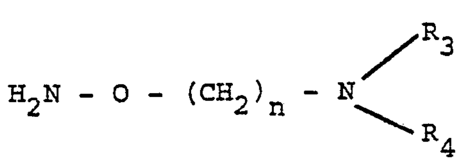

(III)

wobei $R_1$, $R_2$, $R_3$, $R_4$ und n die oben angegebene Bedeutung haben.

8. Verfahren zur Herstellung von Äthern von Oximen der allgemeinen Formel (I) gemäß Anspruch 1, dadurch gekennzeichnet, daß man ein Oxim der allgemeinen Formel (IV)

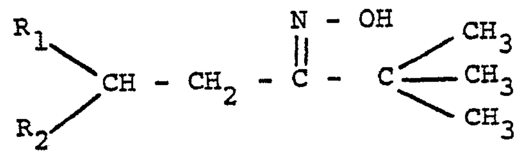

(IV)

mit einem Chloralkylaminderivat der allgemeinen Formel (V)

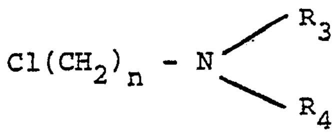

(V)

wobei $R_1$, $R_2$, $R_3$, $R_4$ und n die oben angegebene Bedeutung haben, in einem inerten Lösungsmittel in Gegenwart eines basischen Kondensationsmittels umsetzt.

9. Arzneimittel, dadurch gekennzeichnet, daß es als wirksamen Bestandteil eine physiologisch wirksame Menge mindestens einer Verbindung gemäß einem der Ansprüche 1 bis 6 enthält.

10. Pharmazeutische Zusammensetzung, die ein Arzneimittel gemäß Anspruch 9 in Kombination mit einem pharmazeutisch akzeptablen Vehikel enthält.